# FASCICULE DE BREVET EUROPEEN

(11) **EP 4 210 911 B1**
(45) Date de publication et mention de la délivrance du brevet: **22.10.2025**
(21) Numéro de dépôt: 21782785.6
(22) Date de dépôt: 08.09.2021
(51) Int. Cl.: B25J 9/00, B62D 57/032, B25J 9/16, A61H 1/02, A61H 3/00

(54) **PROCEDE DE MISE EN MOUVEMENT DE L'EXOSQUELETTE**
VERFAHREN ZUM BEWEGEN EINES EXOSKELETTS
METHOD FOR MOVING AN EXOSKELETON

(30) Priorité: 09.09.2020 FR 2009118
(43) Date de publication de la demande: 19.07.2023
(73) Titulaire: Wandercraft, 75004 Paris (FR)
(72) Inventeur: PETRIAUX, Marine, 75004 PARIS (FR); BRUNET, Maxime, 75004 PARIS (FR)
(74) Mandataire: Regimbeau
(86) Numéro de dépôt international: PCT/FR2021/051539
(87) Numéro de publication internationale: WO 2022/053761

(56) Documents cités:
- WO-A1-2019/060791
- US-A1- 2019 159 954

## Description

### DOMAINE TECHNIQUE GENERAL

La présente invention concerne le domaine des robots de type exosquelette.

Plus précisément, elle concerne un procédé de mise en mouvement d'un exosquelette avec un niveau d'assistance variable.

### ETAT DE L'ART

Récemment, sont apparus pour les personnes avec des problèmes de mobilité importants comme les paraplégiques des dispositifs de marche assistée appelés exosquelettes, qui sont des dispositifs robotisés externes que l'opérateur (l'utilisateur humain) vient « enfiler » grâce à un système d'attaches qui lie les mouvements de l'exosquelette de ses propres mouvements. Les exosquelettes de membres inférieurs disposent de plusieurs articulations, généralement au moins au niveau des genoux et des hanches, pour reproduire le mouvement de marche. Des actionneurs permettent de mouvoir ces articulations, qui à leur tour font se mouvoir l'opérateur. Un système d'interface permet à l'opérateur de donner des ordres à l'exosquelette, et un système de commande transforme ces ordres en commande pour les actionneurs. Des capteurs viennent généralement compléter le dispositif.

Un tel exosquelette est connu de US 2019/159954.

Ces exosquelettes constituent une avancée par rapport aux fauteuils roulants, car ils permettent aux opérateurs de se remettre debout et de marcher. Les exosquelettes ne sont plus limités par les roues et peuvent théoriquement évoluer dans la majorité des environnements non plats : les roues, au contraire des jambes, ne permettent pas de franchir des obstacles importants comme des marches, escaliers, obstacles d'une hauteur trop importante, etc.

Outre la récupération de mobilité, les exosquelettes ont un fort intérêt en rééducation, en particulier suite à des accidents neurologiques tels que des AVC.

En général, la rééducation s'effectue grâce au savoir-faire des kinésithérapeutes et des ergothérapeutes. Diverses activités sont pratiquées, pour remuscler les membres mais surtout pour réhabituer le cerveau à donner des signaux électriques correspondant à des ordres cohérents.

L'exosquelette est alors utilisé pour guider les membres des patients pour leur faire suivre une trajectoire prédéterminée. En effet, seuls, ces patients n'effectuent pas les bons mouvements, par exemple en avançant le torse pour lever le genou. L'exosquelette entraîne le mouvement des membres des patients tout en s'opposant aux mouvements non appropriés.

On appelle « assistance » cette force appliquée en faveur des mouvements appropriés et contre les mouvements inappropriés. Mathématiquement, un niveau d'assistance de 100% correspond à un cas dans lequel aucune force n'est nécessaire pour se déplacer le long de la trajectoire prédéterminée, et une assistance de 0% correspond à un cas dans lequel il n'y a aucune force appliquée pour ou contre le mouvement.

On comprend que pour aider les patients à progresser, le niveau d'assistance doit être progressivement réduit pour s'adapter à leurs progrès.

En d'autres termes, il est souhaitable d'avoir un niveau d'assistance variable, ce qui en pratique est extrêmement complexe d'un point de vue mathématique.

La solution basique est de prévoir plusieurs contrôleurs chacun associé à un niveau d'assistance prédéterminé, de sorte à avoir par exemple quatre ou cinq niveaux d'assistance différents entre lesquels on va choisir. On comprend qu'une telle solution n'est pas très modulaire et surtout reste lourde.

Il a par conséquent été proposé dans le document Robot Assisted Gait Training With Active Leg Exoskeleton (ALEX), par Sai K. Banala, Seok Hun Kim, Sunil K. Agrawal, and John P. Scholz, IEEE TRANSACTIONS ON NEURAL SYSTEMS AND REHABILITATION ENGINEERING, VOL. 17, NO. 1, FEBRUARY 2009*,* une solution de niveau d'assistance variable dans laquelle on traite séparément les forces normales Fₙ et les forces tangentielles Ft.

Les forces normales sont utilisées pour ramener le patient vers la trajectoire prévue et s'appliquent uniquement en dehors d'une bande autour de la trajectoire, et augmentent de manière quadratique lorsque le pied s'éloigne de sa position désirée.

Les forces tangentielles sont utilisées aider le patient à réaliser le mouvement et s'appliquent uniquement dans une bande proche de la trajectoire, sont maximales sur la trajectoire, et décroissent linéairement lorsque l'on s'en éloigne.

La variation du niveau d'assistance se fait en modifiant des coefficients appliqués aux forces.

Cette méthode apporte satisfaction, mais reste d'une part limitée à un déplacement planaire contrôlé (ALEX n'est pas un vrai exosquelette mais simplement une prothèse pour une unique jambe fixée à un portique qui la supporte), et d'autre part trop simpliste d'un point de vue mathématique.

En effet on comprend que le calcul des forces dépend uniquement de la distance entre la position courante et la trajectoire et absolument pas du mouvement en lui-même. Il faut à tout moment déterminer le point le plus proche de la position courante sur la trajectoire, et on imagine aisément que si la trajectoire est sensiblement circulaire on risque d'avoir un comportement problématique.

Ainsi, cette solution n'est absolument pas applicable à un vrai exosquelette qui marcherait dans un espace ouvert et qui de surcroît doit supporter son propre poids. Il serait souhaitable de disposer d'une nouvelle solution de mise en mouvement de tout exosquelette avec un niveau d'assistance variable, qui permette de manière fiable et ergonomique la marche de l'exosquelette.

### PRESENTATION DE L'INVENTION

La présente invention se rapporte ainsi selon un premier aspect à un procédé, selon la revendication 1, de mise en mouvement d'un exosquelette bipède recevant un opérateur humain, le procédé comprenant la mise en œuvre par des moyens de traitement de données de l'exosquelette, d'étapes de :
(a) obtention d'une trajectoire élémentaire théorique de l'exosquelette correspondant à un pas ;
(b) paramétrisation de ladite trajectoire élémentaire théorique en fonction d'un unique paramètre, de sorte à définir la trajectoire élémentaire théorique de l'exosquelette comme un guide virtuel à un seul degré de liberté ;
(c) en réponse à des mouvements forcés de l'exosquelette effectués par l'opérateur humain, exécution d'un contrôleur définissant l'évolution d'une position réelle de l'exosquelette en fonction dudit paramètre unique en simulant une liaison ressort-amortisseur entre l'exosquelette et ledit guide virtuel, de sorte à mettre en œuvre une trajectoire élémentaire réelle voisine de ladite trajectoire élémentaire théorique.

Selon des caractéristiques avantageuses et non limitatives :
Le procédé comprend la répétition des étapes (a) à (c) de sorte à faire marcher l'exosquelette par une succession de trajectoires élémentaires réelles correspondantes chacune à un pas.

La trajectoire élémentaire théorique obtenue à l'étape (a) part d'une position initiale, l'étape (c) comprenant la détermination d'une position finale de l'exosquelette à la fin de ladite trajectoire élémentaire réelle, ladite position finale étant utilisée comme position initiale à l'occurrence suivante de l'étape (a).

Ladite liaison ressort-amortisseur entre l'exosquelette et ledit guide virtuel est simulée à l'étape (c) en supposant une force de rappel élastique et une force d'impédance appliquée audit exosquelette.

Ledit contrôleur suppose également qu'une force d'accompagnement tangentielle audit guide virtuel est appliquée à l'exosquelette.

La force de rappel élastique, la force d'impédance et/ou la force d'assistance est fonction d'un niveau d'assistance de l'exosquelette donné.

Ledit contrôleur suppose également qu'une force compensant le poids de l'exosquelette est appliquée à l'exosquelette.

Ladite force de rappel élastique est fonction d'un écart entre la position réelle de l'exosquelette et une position théorique de l'exosquelette le long du guide virtuel ; et ladite force d'impédance est fonction d'un écart entre une dérivée de la position réelle de l'exosquelette et une dérivée de la position théorique de l'exosquelette le long du guide virtuel.

La position de l'exosquelette est définie par un vecteur des positions articulaires des degrés de liberté actionnés de l'exosquelette.

Ledit contrôleur définit l'évolution de la position de l'exosquelette en fonction de l'évolution dudit paramètre unique.

Selon un deuxième aspect, l'invention concerne un exosquelette comprenant des moyens de traitement de données configurés pour mettre en œuvre un procédé selon le premier aspect de mise en mouvement de l'exosquelette.

Selon un troisième aspect, l'invention concerne un système comprenant un serveur et l'exosquelette selon le deuxième aspect, le serveur comprenant des moyens de traitement de données configurés pour générer ladite trajectoire élémentaire théorique et la fournir à l'exosquelette à l'étape (a).

Selon un quatrième et un cinquième aspect, l'invention concerne un produit programme d'ordinateur comprenant des instructions de code pour l'exécution d'un procédé selon le premier aspect de mise en mouvement d'un exosquelette ; et un moyen de stockage lisible par un équipement informatique sur lequel un produit programme d'ordinateur comprend des instructions de code pour l'exécution d'un procédé selon le premier aspect de mise en mouvement d'un exosquelette.

### PRESENTATION DES FIGURES

D'autres caractéristiques et avantages de la présente invention apparaîtront à la lecture de la description qui va suivre d'un mode de réalisation préférentiel. Cette description sera donnée en référence aux dessins annexés dans lesquels :
- La figure 1 est un schéma d'un exosquelette utilisé par les procédés selon l'invention ;
- La figure 2 est un schéma d'une architecture pour la mise en œuvre des procédés selon l'invention ;
- La figure 3 est un diagramme illustrant un mode de réalisation préféré du procédé selon l'invention.

### DESCRIPTION DETAILLEE

### Architecture

La présente invention propose un procédé de mise en mouvement d'un exosquelette 1.

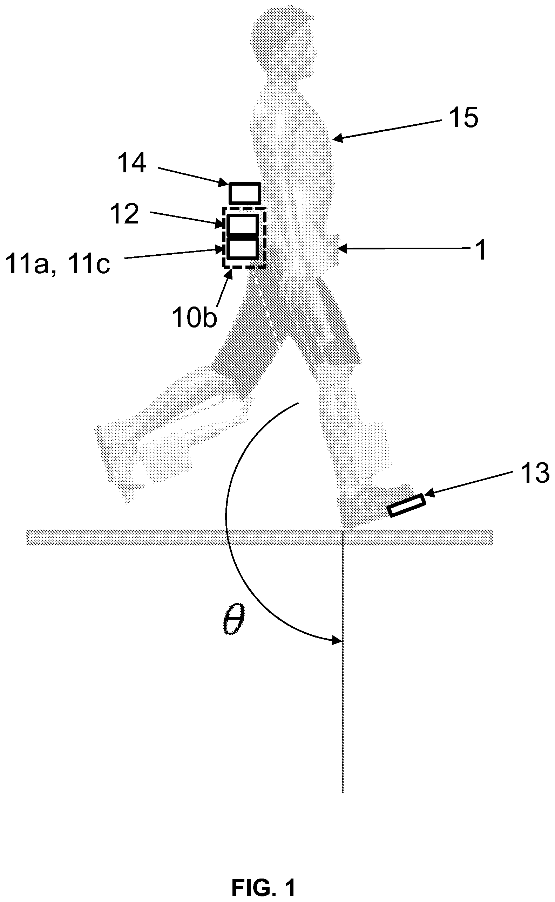

En référence à la **Figure 1****,** ledit exosquelette 1 est un système mécanique articulé de type dispositif robotisé bipède, actionné et commandé, pourvu de deux jambes, accueillant plus précisément un opérateur humain présentant ses membres inférieurs chacun solidaires d'une jambe de l'exosquelette 1 (notamment grâce à des sangles). Il peut ainsi être un robot plus ou moins humanoïde. Par « marche », on entend ici la mise en mouvement du dispositif robotisé 1, qui se traduit en pratique par en appui alternatif sur les jambes, en position debout, de sorte à produire un déplacement. Comme l'on verra plus loin, on suppose qu'un mouvement de l'exosquelette est composé d'une séquence de pas, chaque pas voyant un pied se décoller du sol puis se reposer, avant inversion des rôles (i.e. une alternance de pas du pied gauche et du pied droit).

L'exosquelette 1 présente une pluralité de degrés de liberté, c'est-à-dire d'articulations déformables (généralement via une rotation) c'est-à-dire mobiles les unes par rapport aux autres, qui sont chacun soit « actionné », soit « non-actionné ».

Un degré de liberté actionné désigne une articulation pourvue d'un actionneur commandé par des moyens de traitement de données 11c, c'est-à-dire que ce degré de liberté est contrôlé et que l'on peut agir dessus. Au contraire un degré de liberté non actionné désigne une articulation dépourvue d'actionneur, c'est-à-dire que ce degré de liberté suit sa propre dynamique et que les moyens de traitement de données 11 n'ont pas de contrôle direct dessus (mais a priori un contrôle indirect via les autres degrés de liberté actionnés).

Le présent exosquelette comprend naturellement au moins deux degrés de liberté actionnés, préférentiellement une pluralité.

Dans le présent cas, l'exosquelette 1 présente comme expliqué un niveau d'assistance variable, c'est-à-dire que l'opérateur peut de par ses mouvements « actionner » de lui-même les degrés de liberté dans la mesure où le niveau d'assistance le permet, i.e. modifier de lui-même l'orientation d'une articulation, par exemple plier le genou. A ce titre, les actionneurs sont avantageusement également des capteurs, en ce qu'ils sont capables de faire remonter aux moyens de traitement de données 11c leur position de sorte à pouvoir détecter l'actionnement « forcé » d'un degré de liberté par l'opérateur.

Les moyens de traitement de données 11c désignent un équipement informatique (typiquement un processeur, soit externe si l'exosquelette 1 est « télécommandé » mais préférentiellement embarqué dans l'exosquelette 1, voir plus loin) adapté pour traiter des instructions et générer des commandes à destination des différents actionneurs. Ces derniers peuvent être électriques, hydrauliques, etc.

La présente demande ne sera limitée à aucune architecture d'exosquelette 1, et on prendra l'exemple tel que décrit dans les demandes WO2015140352 et WO2015140353.

Ainsi, de façon préférée et conformément à ces demandes, l'exosquelette 1 comprend sur chaque jambe une structure de pied comprenant un plan de support sur lequel un pied d'une jambe de la personne portant l'exosquelette peut venir en appui.

Ce plan de support comprend une plate-forme avant et une plate-forme arrière, telles qu'une liaison pivot pied relie la plate-forme avant à la plate-forme arrière, en constituant un degré de liberté non actionné.

L'homme du métier saura toutefois adapter le présent procédé à toute autre architecture mécanique.

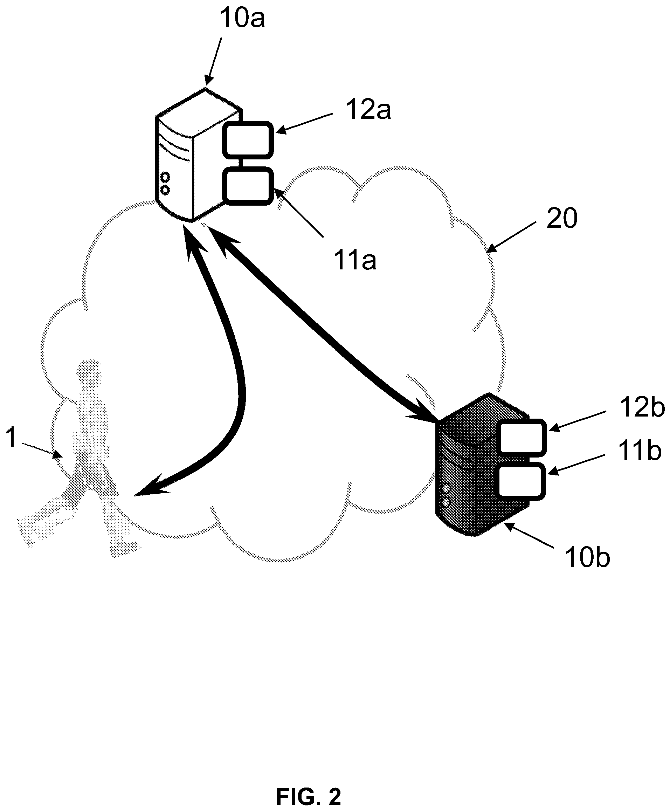

Selon un mode de réalisation préféré, le présent procédé de génération de trajectoire et de marche peuvent impliquer un premier voire un deuxième serveur 10a, 10b au sein d'une architecture telle que représentée par la **Figure 2****.**

Le premier serveur 10a est un serveur de génération de trajectoire, et le deuxième serveur 10b est un éventuel serveur d'apprentissage.

En effet, la génération d'une trajectoire de l'exosquelette 1 peut utiliser un réseau de neurone, en particulier de type « à propagation avant » (FNN, « Feedforward Neural Network »), comme cela est proposé dans la demande FR1910649. Le deuxième serveur 10b est alors un serveur pour la mise en œuvre d'un procédé d'apprentissage de paramètres dudit réseau de neurones. A noter que le présent procédé n'est pas limité à l'utilisation d'un réseau de neurones, et on pourra utiliser toute technique connue de génération de la trajectoire dans sa globalité, voire plus loin.

Dans tous les cas, il est tout à fait possible que ces deux serveurs soient confondus, mais en pratique le deuxième serveur 10b est le plus souvent un serveur distant alors que le premier serveur 10a peut être embarqué par l'exosquelette 1 pour fonctionnement en temps réel, comme cela est représenté par la figure 2. Selon un mode de réalisation préféré, le premier serveur 10a met en œuvre le procédé de génération d'une trajectoire de l'exosquelette 1 grâce à un réseau de neurones utilisant les paramètres récupérés depuis le second serveur 10b, et l'exosquelette 1 applique normalement directement ladite trajectoire générée in situ pour se mettre en mouvement.

Chacun de ces serveurs 10a, 10b est typiquement un équipement informatique relié à un réseau étendu 20 tel que le réseau internet pour l'échange des données, même si en pratique une fois le réseau de neurone appris et embarqué sur le deuxième serveur 10b la communication peut être interrompue, du moins par intermittence. Chacun comprend des moyens de traitement de données 11a, 11b de type processeur (en particulier les moyens de traitement de données 11b du deuxième serveur ont une forte puissance de calcul, car l'apprentissage est long et complexe par rapport à la simple utilisation du réseau de neurones appris), et le cas échéant des moyens de stockage de données 12a, 12b telle qu'une mémoire informatique, par exemple un disque dur. Dans le cas d'une génération de trajectoire par réseau de neurones, une base de données d'apprentissage peut être stockée par la mémoire 12b du deuxième serveur 10b.

On comprendra qu'il peut y avoir une pluralité d'exosquelettes 1 embarquant chacun leur premier serveur 10a (qui peut alors être de puissance et d'encombrement limité, dans la mesure où il ne génère de trajectoires que pour l'exosquelette 1 auquel il est dédié), ou bien une pluralité d'exosquelettes 1 connectés chacun à un premier serveur 10a plus puissant et éventuellement confondu avec le second serveur 10b (et ayant la capacité de générer des trajectoires à la volée pour tous les exosquelettes 1).

### Trajectoire

Comme expliqué, on entend classiquement par « trajectoire » de l'exosquelette les évolutions de chaque degré de liberté (en particulier actionné) exprimées en fonction du temps ou d'une variable de phase. Dans la suite de la présente description, par « position » de l'exosquelette 1 on entendra les positions articulaires des degrés de liberté actionnés, qui sont avantageusement au nombre de six par jambe, soit une position définie par un vecteur de dimension 12, même si l'on pourrait par exemple prendre la position cartésienne d'un point caractéristique de l'exosquelette, par exemple son CMP (on a lors un vecteur de dimension 6 : 3 positions plus 3 orientations selon les 3 axes). A noter que la solution à 12 degrés de liberté est préférable car il y a de la redondance et donc elle force l'opérateur à adopter une bonne coordination articulaire.

Par ailleurs, on sait définir un mouvement « complexe » comme une séquence de trajectoires dites « élémentaires » le cas échéant entrecoupées de transitions. Par trajectoire élémentaire, on entend toute trajectoire correspondant à un pas, i.e. appliquée sur la durée du pas de sorte que partant d'un état initial de l'exosquelette 1 au début d'un pas (moment de contact du pied), on retourne au même état au début du pas suivant. A noter qu'on a une alternance de pas gauche et pas droit, de sorte que l'état à la fin d'un pas est « symétrisé » (ce n'est plus le même pied devant), et il faut techniquement deux pas pour retourner exactement dans le même état (même pied devant). On appelle trajectoire périodique une succession stable de trajectoires élémentaires permettant la marche.

Cela englobe toute marche à plat, mais également sur rampe, une montée ou descente d'un escalier, etc.).

Une trajectoire élémentaire est associée à une marche donnée de l'exosquelette 1 (une marche étant définie par un n-uplet de paramètres de marche), et permet de maintenir cette marche de manière stable et faisable (i.e. comme on le verra respecte toutes les contraintes d'un problème d'optimisation et minimise autant que possible une fonction de coût). Comme expliqué, lesdits paramètres de marche correspondent à des « caractéristiques » de la façon de marcher, telles que la longueur des pas, la fréquence de marche et l'inclinaison du buste, mais également hauteur des marches en cas de franchissement d'escaliers, l'angle de rotation instantané pour les mouvements courbes ; et également à des caractéristiques morphologiques de l'opérateur (un sous-groupe des paramètres de marche dits paramètres patient) telles que sa taille, son poids, la longueurs des cuisses ou des tibia, la position du centre de masse (valeur du décalage vers l'avant) et le débattement latéral du buste dans le cadre d'activité de rééducation.

Lesdites « contraintes » d'une marche évoquées ci-avant peuvent être variées et dépendre du type de marche souhaitée, par exemple une marche « pied plat », ou « avec déroulé », etc. Le présent procédé ne sera limité à aucun type de marche souhaité.

Les éventuelles transitions correspondent à des changements de marche, i.e. des variations de valeurs desdits paramètres de marche (par exemple une augmentation de la longueur de pas) : connaissant un jeu initial de paramètres de marche et un jeu final de paramètres de marche, et donc une trajectoire périodique initiale (associée au jeu initial de paramètres de marche) et une trajectoire périodique finale (associée au jeu final de paramètres de marche), ladite transition est un fragment de trajectoire permettant de passer de la trajectoire périodique initiale à la trajectoire finale. On note qu'il faut y avoir des transitions « initiale » ou « finale », correspondant à des démarrage et fin du mouvement.

### Procédé

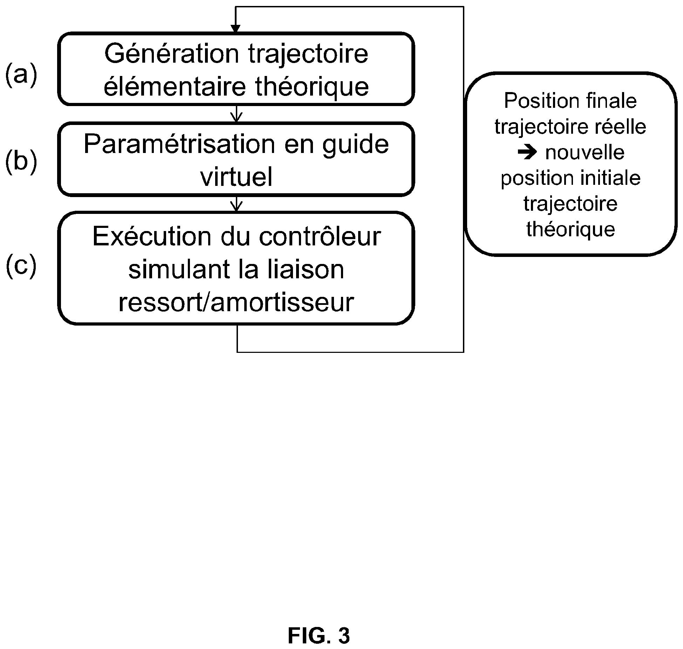

En référence à la **Figure 3****,** ledit procédé de mise en mouvement de l'exosquelette 1, mis en œuvre par les moyens de traitement de données 11c embarqués, commence par une étape (a) d'obtention d'une trajectoire élémentaire théorique de l'exosquelette correspondant à un pas. Cette étape peut comprendre l'obtention préalable d'au moins un n-uplet de paramètres de marche définissant une marche donnée de l'exosquelette 1, voire une séquence de n-uplets de paramètres de marche progressivement (par exemple du fait de nouvelles commandes de la part de l'opérateur de l'exosquelette ou d'un thérapeute, notamment si l'exercice évolue).

A noter que l'obtention peut comme expliqué impliquer directement la génération de la trajectoire par l'exosquelette 1 (si par exemple il embarque le serveur 10a) ou bien la simple réception de la trajectoire par le réseau 20. A ce titre les moyens 11c peuvent fournir au server 10a externe les paramètres de marche, et récupérer en retour la tractoire.

A noter que l'on génère le plus souvent directement une trajectoire périodique théorique qui va plus loin qu'un seul pas, la trajectoire élémentaire théorique en est juste un fragment.

Préférentiellement un niveau d'assistance de l'exosquelette est défini. Comme expliqué, il s'agit avantageusement d'une valeur entre 0 et 1, 0 désignant une assistance nulle et 1 désignant une assistance totale, même si on pourra utiliser toute échelle, une pluralité de niveaux prédéfinis, etc. A nouveau le niveau d'assistance peut varier. On supposera seulement que le niveau d'assistance est tel que des mouvements forcés de l'exosquelette 1 effectués par l'opérateur humain sont en pratique possibles, l'exosquelette 1 ne les bloquant pas.

A noter que l'opérateur peut être muni comme expliqué d'un gilet de capteurs 15 permettant de détecter la configuration de son buste (orientation de celui-ci). La direction dans laquelle l'opérateur oriente son buste est celle dans laquelle il souhaite marcher et la vitesse est donnée par l'intensité avec laquelle il met son buste en avant (à quel point il se penche). La requête de démarrage peut correspondre à l'appui par l'opérateur sur un bouton (ou une posture particulière) signifiant son intention de se mettre en marche et donc ordonnant aux moyens de traitement de données de déterminer lesdits paramètres. Certains paramètres tels que l'angle de rotation instantané ou la hauteur des marches en cas de franchissement d'escaliers peuvent être prédéterminés ou obtenus au moyen d'autres capteurs 13, 14.

Pour la génération de la trajectoire à proprement parler, on ne sera limité à aucune technique connue. On connait notamment comme expliqué des outils d'optimisation, capables notamment de générer une trajectoire donnée en fonction des contraintes et paramètres de marches choisis. Par exemple, dans le cas de trajectoires HZD, le problème de la génération de trajectoires est formulé sous la forme d'un problème de contrôle optimal qui peut être résolu de manière préférée par un algorithme dit de collocation directe, voir le document Omar Harib et al., Feedback Control of an Exoskeleton for Paraplegics Toward Robustly Stable Hands-free Dynamic Walking.

On pourra alternativement comme expliqué également utiliser un réseau de neurones entraîné sur une base de données de trajectoires d'apprentissage.

Dans tous les cas, on supposera défini une position initiale de l'exosquelette 1 correspondant à sa position au début du pas.

La trajectoire élémentaire générée est dite « théorique », par opposition à une trajectoire « réelle ». En effet, dans un fonctionnement classique de l'exosquelette 1 sans utilisation en rééducation, on pourrait simplement appliquer la trajectoire théorique et l'exosquelette marcherait automatiquement conformément à cette trajectoire.

Ici, dans le but d'entraîner l'opérateur, on répète que des mouvements forcés de l'exosquelette 1 effectués par l'opérateur humain sont en pratique possibles, de sorte que la trajectoire qui est en pratique effectuée par l'exosquelette 1 (la trajectoire réelle) ne correspond jamais exactement à la trajectoire théorique prévue, même si comme on verra le présent procédé fait qu'elles resteront proches. On comprend donc que ladite trajectoire réelle est conforme aux mouvements de l'opérateur. Pour reformuler encore, la trajectoire réelle peut être exécutée à une vitesse librement choisie par l'opérateur humain, selon ses mouvements.

Ensuite, dans une étape (b), le procédé comprend la paramétrisation de ladite trajectoire élémentaire théorique en fonction d'un unique paramètre, de sorte à définir la trajectoire élémentaire théorique de l'exosquelette 1 comme un guide virtuel à un seul degré de liberté.

L'idée des guides virtuels est bien connue en cobotique, et décrite par exemple dans le document Iterative Virtual Guides Programming for Human-Robot Comanipulation, Susana Sanchez Restrepo, Gennaro Raiola, Pauline Chevalier, Xavier Lamy and Daniel Sidobre. Ces guides virtuels sont par exemple des « barrières » limitant le mouvement de robots effecteurs, en particulier pour des raisons de sécurité.

Par exemple, le robot peut être un bras robotisé terminé par une scie circulaire manipulée par un opérateur humain, la trajectoire théorique est une trajectoire de découpe, et le guide virtuel empêche que l'opérateur se blesse s'il s'éloigne trop de la trajectoire.

Comme l'on va voir, le guide virtuel constitue s'adapte astucieusement problème de l'assistance avec un exosquelette.

Mathématiquement on définit un premier couple {xᵥₘ ; ẋᵥₘ} de la position théorique de l'exosquelette 1 et de la dérivée de la position théorique de l'exosquelette 1 (lorsque la trajectoire élémentaire théorique est parcourue), et un deuxième couple {x ; x} de la position réelle de l'exosquelette 1 et de la dérivée de la position réelle de l'exosquelette 1 (lorsque la trajectoire élémentaire réelle est parcourue). Ici « vm » signifie virtual mechanism car on peut voir l'exosquelette 1 suivant la trajectoire théorique comme un « mécanisme virtuel » glissant le long du guide virtuel. En d'autres termes, xᵥₘ est la position théorique de l'exosquelette 1 le long du guide virtuel. On répète que tous les objets x, xᵥₘ, X et ẋᵥₘ sont des vecteurs, préférentiellement de dimension 12.

Dans la mesure où, dans l'espace de positions de l'exosquelettes, le guide virtuel forme un objet de dimension 1 (une courbe), on peut la paramétriser avec un unique paramètre noté sᵥₘ. Ce paramètre peut être le temps, mais préférentiellement c'est l'abscisse curviligne le long du guide virtuel par exemple en une valeur 0 lorsque l'exosquelette est dans la position initiale, et une valeur 1 lorsqu'il est dans une position finale correspondant à la fin du pas (la jambe levée atteint le sol).

On peut poser xᵥₘ=Lₛ(sᵥₘ) et ẋᵥₘ=Jₛ(ṡᵥₘ), où Lₛ est le modèle de paramétrisation et Jₛ est le jacobien du mécanisme virtuel, de sorte à définir son modèle cinématique. L'homme du métier saura mettre un œuvre cette paramétrisation, par exemple en s'inspirant du document Iterative Virtual Guides Programming for Human-Robot Comanipulation, Susana Sanchez Restrepo, Gennaro Raiola, Pauline Chevalier, Xavier Lamy and Daniel Sidobre*.*

Dans une étape (c) principale, en réponse à des mouvements forcés de l'exosquelette 1 effectués par l'opérateur humain (i.e. opérateur va tenter de lui-même de mettre en œuvre l'exosquelette 1 conformément à la trajectoire prévue, en particulier sous le contrôle du thérapeute), les moyens 11c exécutent un contrôleur définissant l'évolution d'une position réelle de l'exosquelette 1 en fonction dudit paramètre unique en simulant une liaison ressort-amortisseur entre l'exosquelette 1 et ledit guide virtuel, de sorte à mettre en œuvre une trajectoire élémentaire réelle voisine de ladite trajectoire élémentaire théorique.

Ce contrôleur peut naturellement être préparé à l'avance et chargé sur l'exosquelette 1.

La « liaison ressort-amortisseur » évoquée est une analogie physique illustrant l'idée que l'exosquelette 1 serait attachée au guide virtuel par un ressort et un amortisseur en parallèle : tant que la trajectoire réelle mise en œuvre par l'utilisateur est proche de la trajectoire théorique rien ne se passe, mais dès qu'il s'en éloigne les forces du ressort et de l'amortisseur entrent en compte.

De manière préférée, ladite liaison ressort-amortisseur entre l'exosquelette 1 et ledit guide virtuel est simulée à l'étape (c) en supposant une force de rappel élastique (le ressort) et une force d'impédance (l'amortisseur) appliquée audit exosquelette 1.

En particulier, ladite force de rappel élastique est fonction d'un écart entre la position réelle x de l'exosquelette 1 et la position théorique xᵥₘ de l'exosquelette 1 le long du guide virtuel (avantageusement selon la formule F₁=K(xᵥₘ - x), où K est un coefficient de raideur qui peut être fonction du niveau d'assistance) et ladite force d'impédance est fonction d'un écart entre la dérivée x de la position réelle de l'exosquelette 1 et la dérivée ẋᵥₘ de la position théorique de l'exosquelette 1 le long du guide virtuel (avantageusement selon la formule F₂=B(ẋᵥₘ - x), où B est un coefficient d'amortissement qui peut être également fonction du niveau d'assistance).

L'idée est de lister les forcer appliquées à l'exosquelette 1, et de déterminer sa dynamique au premier ordre par rapport audit paramètre unique sᵥₘ, de sorte que ledit contrôleur (en particulier sous la forme d'un contrôleur PD) définisse l'évolution de la position réelle x de l'exosquelette 1 en fonction de l'évolution dudit paramètre unique.

On rajoute avantageusement :
- une force F₃ d'accompagnement tangentielle audit guide virtuel et d'intensité fonction dudit niveau d'assistance (par exemple F₃=Nẋᵥₘ. A noter qu'on peut davantage compenser certains degrés de libertés par exemple les hanches frontales et transverses sont assez peu mobilisables et on peut les assister davantage pour qu'elles ne soient pas bloquantes, i.e. N peut être une matrice diagonale avec des coefficients variés potentiellement également fonction du niveau d'assistance et/ou du poids de l'opérateur) ;
- une force F₄ compensant le poids de l'exosquelette (F₄= -P)

On a alors Jₛ^{T}(F₁+F₂+F₃+F₄)=0, équation qu'on peut résoudre vis à vis de ṡᵥₘ, puis intégrer, de sorte à connaître l'évolution de la position réelle x de l'exosquelette 1 en fonction de l'évolution dudit paramètre unique sᵥₘ.

En résumé, l'opérateur est assisté par F₃, n'a pas à supporter le poids de l'exosquelette grâce à F₄, et est ramené vers la trajectoire théorique par F₁ et ce de façon souple par F₂.

Par rapport à la solution ALEX évoquée dans l'introduction, il n'y a pas de distance à calculer et on a un procédé universel.

### Enchainement de pas

Les étapes (a) à (c) peuvent être répétées de sorte à faire marcher l'exosquelette 1 par une succession de trajectoires élémentaires réelles correspondantes chacune à un pas.

Il faut garder en tête que la trajectoire réelle est différente de la trajectoire théorique, de sorte qu'il faut adapter la trajectoire suivante au pas qui vient de se dérouler.

Ainsi, la trajectoire élémentaire théorique obtenue à l'étape (a) part d'une position initiale, l'étape (c) comprenant avantageusement la détermination d'une position finale de l'exosquelette 1 à la fin de ladite trajectoire élémentaire réelle, ladite position finale étant utilisée comme position initiale à l'occurrence suivante de l'étape (a).

Comme expliqué, généralement on génère une trajectoire périodique complète (constituée d'une suite de trajectoires élémentaires), de sorte que la nouvelle occurrence de l'étape (a) consiste à modifier la trajectoire périodique (pour la prochaine trajectoire élémentaire).

En d'autres termes, il y a donc une interpolation entre la position de l'exosquelette 1 au moment de l'impact et le pas suivant, afin d'éviter un saut des consignes. L'interpolation est réalisée au moment de l'impact, et modifie le début de la trajectoire du pas suivant. Le guide virtuel est alors « déplacé » (nouvelle occurrence de l'étape (b)) pour correspondre à la position actuelle et pouvoir démarrer le pas suivant sur de bonnes bases.

### Equipements et système

Selon un deuxième aspect, l'invention concerne l'exosquelette 1, pour la mise en œuvre du procédé selon le premier aspect, et selon un troisième aspect le système comprenant l'exosquelette ainsi qu'un éventuel serveur 10a, possiblement confondus.

L'exosquelette 1 comprend des moyens de traitement de données 11c configurés pour la mise en œuvre du procédé selon le deuxième aspect, ainsi que si nécessaire des moyens de stockage de données 12 (en particulier ceux du premier serveur 10a), des moyens de mesure inertielle 14 (centrale à inertie), des moyens pour détecter l'impact des pieds au sol 13 (capteurs de contact ou éventuellement capteurs de pression), et/ou un gilet de capteurs 15.

Il présente une pluralité de degrés de liberté dont au moins un degré de liberté actionné par un actionneur commandé par les moyens de traitement de données 11c pour l'exécution dudit contrôleur.

Le premier serveur 10a comprend des moyens de traitement de données 11a pour générer ladite trajectoire élémentaire théorique et la fournir à l'exosquelette à l'étape (a), en particulier sur réception de la position initiale de l'exosquelette 1 au démarrage pas et des éventuels paramètres de marche

### Produit programme d'ordinateur

Selon un troisième et un quatrième aspects, l'invention concerne un produit programme d'ordinateur comprenant des instructions de code pour l'exécution (sur les moyens de traitement 11c), d'un procédé selon le premier aspect de de mise en mouvement d'un exosquelette 1, ainsi que des moyens de stockage lisibles par un équipement informatique sur lequel on trouve ce produit programme d'ordinateur.

## Revendications

1. Procédé de mise en mouvement d'un exosquelette (1) bipède recevant un opérateur humain, le procédé comprenant la mise en œuvre par des moyens de traitement de données (11c) de l'exosquelette (1), d'une étape de :
(a) obtention d'une trajectoire élémentaire théorique de l'exosquelette (1) correspondant à un pas ;
**caractérisé en ce que** le procédé comprend en outre la mise en œuvre, par les moyens de traitement de données (11c) de l'exosquelette (1), d'étapes de :
(b) paramétrisation de ladite trajectoire élémentaire théorique en fonction d'un unique paramètre, de sorte à définir la trajectoire élémentaire théorique de l'exosquelette (1) comme un guide virtuel à un seul degré de liberté ;
(c) en réponse à des mouvements forcés de l'exosquelette (1) effectués par l'opérateur humain, exécution d'un contrôleur définissant l'évolution d'une position réelle de l'exosquelette (1) en fonction dudit paramètre unique en simulant une liaison ressort-amortisseur entre l'exosquelette (1) et ledit guide virtuel, de sorte à mettre en œuvre une trajectoire élémentaire réelle voisine de ladite trajectoire élémentaire théorique.

2. Procédé selon la revendication 1, comprenant la répétition des étapes (a) à (c) de sorte à faire marcher l'exosquelette (1) par une succession de trajectoires élémentaires réelles correspondantes chacune à un pas.

3. Procédé selon la revendication 2, dans lequel la trajectoire élémentaire théorique obtenue à l'étape (a) part d'une position initiale, l'étape (c) comprenant la détermination d'une position finale de l'exosquelette (1) à la fin de ladite trajectoire élémentaire réelle, ladite position finale étant utilisée comme position initiale à l'occurrence suivante de l'étape (a).

4. Procédé selon l'une des revendications 1 à 3, dans lequel ladite liaison ressort-amortisseur entre l'exosquelette (1) et ledit guide virtuel est simulée à l'étape (c) en supposant une force de rappel élastique et une force d'impédance appliquée audit exosquelette (1).

5. Procédé selon la revendication 4, dans lequel ledit contrôleur suppose également qu'une force d'accompagnement tangentielle audit guide virtuel est appliquée à l'exosquelette.

6. Procédé selon la revendication 5, dans lequel la force de rappel élastique, la force d'impédance et/ou la force d'assistance est fonction d'un niveau d'assistance de l'exosquelette (1) donné.

7. Procédé selon l'une des revendications 4 à 6, dans lequel ledit contrôleur suppose également qu'une force compensant le poids de l'exosquelette (1) est appliquée à l'exosquelette.

8. Procédé selon l'une des revendications 4 à 7, dans lequel ladite force de rappel élastique est fonction d'un écart entre la position réelle de l'exosquelette (1) et une position théorique de l'exosquelette (1) le long du guide virtuel ; et ladite force d'impédance est fonction d'un écart entre une dérivée de la position réelle de l'exosquelette (1) et une dérivée de la position théorique de l'exosquelette (1) le long du guide virtuel.

9. Procédé selon l'une des revendications 1 à 8, dans lequel la position de l'exosquelette (1) est définie par un vecteur des positions articulaires des degrés de liberté actionnés de l'exosquelette (1).

10. Procédé selon l'une des revendications 1 à 9, dans lequel ledit contrôleur définit l'évolution de la position de l'exosquelette en fonction de l'évolution dudit paramètre unique.

11. Exosquelette (1) comprenant des moyens de traitement de données (11c) configurés pour mettre en œuvre un procédé selon l'une des revendications 1 à 10 de mise en mouvement de l'exosquelette (1).

12. Système comprenant un serveur (10a) et l'exosquelette (1) selon la revendication 11, le serveur (10a) comprenant des moyens de traitement de données (11a) configurés pour générer ladite trajectoire élémentaire théorique et la fournir à l'exosquelette à l'étape (a).

13. Produit programme d'ordinateur comprenant des instructions de code pour l'exécution d'un procédé selon l'une des revendications 1 à 10 de mise en mouvement d'un exosquelette (1), lorsque ledit programme est exécuté sur un ordinateur.

14. Moyen de stockage lisible par un équipement informatique sur lequel un produit programme d'ordinateur comprend des instructions de code pour l'exécution d'un procédé selon l'une des revendications 1 à 10 de mise en mouvement d'un exosquelette (1).

## Patentansprüche

1. Verfahren zum Bewegen eines zweibeinigen Exoskeletts (1), das einen menschlichen Bediener aufnimmt, wobei das Verfahren die Durchführung des folgenden Schritts durch Datenverarbeitungsmittel (11c) des Exoskeletts (1) umfasst:
(a) Erhalten einer theoretischen Elementarbahn des Exoskeletts (1), die einem Schritt entspricht;
**dadurch gekennzeichnet, dass** das Verfahren ferner die Durchführung der folgenden Schritte durch die Datenverarbeitungsmittel (11c) des Exoskeletts (1) umfasst:
(b) Parametrisieren der theoretischen Elementarbahn in Abhängigkeit von einem einzigen Parameter, um die theoretische Elementarbahn des Exoskeletts (1) als virtuelle Führung mit einem einzigen Freiheitsgrad zu definieren;
(c) als Reaktion auf vom menschlichen Bediener ausgeführte Zwangsbewegungen des Exoskeletts (1), Ausführen eines Controllers, der die Entwicklung einer tatsächlichen Position des Exoskeletts (1) in Abhängigkeit von dem einzigen Parameter definiert, indem eine Feder-Dämpfer-Verbindung zwischen dem Exoskelett (1) und der virtuellen Führung simuliert wird, um eine tatsächliche Elementarbahn nahe der theoretischen Elementarbahn auszuführen.

2. Verfahren nach Anspruch 1, umfassend die Wiederholung der Schritte (a) bis (c), um das Exoskelett (1) durch eine Abfolge von tatsächlichen Elementarbahnen, die jeweils einem Schritt entsprechen, zum Laufen zu bringen.

3. Verfahren nach Anspruch 2, wobei die in Schritt (a) erhaltene theoretische Elementarbahn von einer Ausgangsposition ausgeht, wobei Schritt (c) die Bestimmung einer Endposition des Exoskeletts (1) am Ende der tatsächlichen Elementarbahn umfasst, wobei die Endposition als Ausgangsposition für den nächsten Schritt (a) verwendet wird.

4. Verfahren nach einem der Ansprüche 1 bis 3, wobei die Feder-Dämpfer-Verbindung zwischen dem Exoskelett (1) und der virtuellen Führung in Schritt (c) unter der Annahme einer auf das Exoskelett (1) ausgeübten elastischen Rückstellkraft und einer auf das Exoskelett (1) ausgeübten Impedanzkraft simuliert wird.

5. Verfahren nach Anspruch 4, wobei der Controller ebenfalls davon ausgeht, dass eine tangential zur virtuellen Führung wirkende Unterstützungskraft auf das Exoskelett ausgeübt wird.

6. Verfahren nach Anspruch 5, wobei die elastische Rückstellkraft, die Impedanzkraft und/oder die Unterstützungskraft von einem gegebenen Unterstützungsgrad des Exoskeletts (1) abhängt.

7. Verfahren nach einem der Ansprüche 4 bis 6, wobei der Controller ebenfalls davon ausgeht, dass eine das Gewicht des Exoskeletts (1) ausgleichende Kraft auf das Exoskelett ausgeübt wird.

8. Verfahren nach einem der Ansprüche 4 bis 7, wobei die elastische Rückstellkraft von einer Abweichung zwischen der tatsächlichen Position des Exoskeletts (1) und einer theoretischen Position des Exoskeletts (1) entlang der virtuellen Führung abhängt; und die Impedanzkraft von einer Abweichung zwischen einer Ableitung der tatsächlichen Position des Exoskeletts (1) und einer Ableitung der theoretischen Position des Exoskeletts (1) entlang der virtuellen Führung abhängt.

9. Verfahren nach einem der Ansprüche 1 bis 8, wobei die Position des Exoskeletts (1) von einem Vektor der Gelenkpositionen der betätigten Freiheitsgrade des Exoskeletts (1) definiert wird.

10. Verfahren nach einem der Ansprüche 1 bis 9, wobei der Controller die Entwicklung der Position des Exoskeletts in Abhängigkeit von der Entwicklung des einzigen Parameters definiert.

11. Exoskelett (1), umfassend Datenverarbeitungsmittel (11c), die ausgelegt sind, um ein Verfahren nach einem der Ansprüche 1 bis 10 zum Bewegen des Exoskeletts (1) durchzuführen.

12. System, umfassend einen Server (10a) und das Exoskelett (1) nach Anspruch 11, wobei der Server (10a) Datenverarbeitungsmittel (11a) umfasst, die ausgelegt sind, um die theoretische Elementarbahn zu erzeugen und sie dem Exoskelett in Schritt (a) bereitzustellen.

13. Computerprogrammprodukt, umfassend Codeanweisungen zur Ausführung eines Verfahrens nach einem der Ansprüche 1 bis 10 zum Bewegen eines Exoskeletts (1), wenn das Programm auf einem Computer ausgeführt wird.

14. Speichermedium, das von einer IT-Ausrüstung lesbar ist, auf dem ein Computerprogrammprodukt Codeanweisungen zur Ausführung eines Verfahrens nach einem der Ansprüche 1 bis 10 zum Bewegen eines Exoskeletts (1) umfasst.

## Claims

1. A method for setting in motion a bipedal exoskeleton (1) receiving a human operator, the method comprising the implementation, by data processing means (11c) of the exoskeleton (1), of a step of:
(a) obtaining a theoretical elementary trajectory of the exoskeleton (1) corresponding to a step;
**characterized in that** the method further comprises the implementation, by the data processing means (11c) of the exoskeleton (1), of steps of:
(b) parametrizing said theoretical elementary trajectory as a function of a single parameter, so as to define the theoretical elementary trajectory of the exoskeleton (1) as a virtual guide with a single degree of freedom;
(c) in response to forced movements of the exoskeleton (1) performed by the human operator, executing a controller defining the evolution of an actual position of the exoskeleton (1) as a function of said single parameter by simulating a spring-damper connection between the exoskeleton (1) and said virtual guide, so as to implement an actual elementary trajectory in the vicinity of said theoretical elementary trajectory.

2. The method according to claim 1, comprising repeating steps (a) to (c) so as to make the exoskeleton (1) walk through a succession of actual elementary trajectories each corresponding to a step.

3. The method according to claim 2, wherein the theoretical elementary trajectory obtained in step (a) starts from an initial position, step (c) comprising determining a final position of the exoskeleton (1) at the end of said actual elementary trajectory, said final position being used as initial position on the next occurrence of step (a).

4. The method according to any of claims 1 to 3, wherein said spring-damper connection between the exoskeleton (1) and said virtual guide is simulated in step (c) by assuming an elastic restoring force and an impedance force applied to said exoskeleton (1).

5. The method according to claim 4, wherein said controller also assumes that an accompanying force tangential to said virtual guide is applied to the exoskeleton.

6. The method according to claim 5, wherein the elastic restoring force, the impedance force and/or the assistance force is a function of a given level of assistance of the exoskeleton (1).

7. The method according to any of claims 4 to 6, wherein said controller also assumes that a force compensating for the weight of the exoskeleton (1) is applied to the exoskeleton.

8. The method according to any of claims 4 to 7, wherein said elastic restoring force is a function of a deviation between the actual position of the exoskeleton (1) and a theoretical position of the exoskeleton (1) along the virtual guide; and said impedance force is a function of a deviation between a derivative of the actual position of the exoskeleton (1) and a derivative of the theoretical position of the exoskeleton (1) along the virtual guide.

9. The method according to any of claims 1 to 8, wherein the position of the exoskeleton (1) is defined by a vector of the joint positions of the actuated degrees of freedom of the exoskeleton (1).

10. The method according to any of claims 1 to 9, wherein said controller defines the evolution of the position of the exoskeleton as a function of the evolution of said single parameter.

11. An exoskeleton (1) comprising data processing means (11c) configured to implement a method according to any of claims 1 to 10 for setting in motion the exoskeleton (1).

12. A system comprising a server (10a) and the exoskeleton (1) according to claim 11, the server (10a) comprising data processing means (11a) configured to generate said theoretical elementary trajectory and provide it to the exoskeleton in step (a).

13. A computer program product comprising code instructions for the execution of a method according to any of claims 1 to 10 for setting in motion an exoskeleton (1), when said program is executed on a computer.

14. A storage means readable by a computer equipment on which a computer program product comprises code instructions for the execution of a method according to any of claims 1 to 10 for setting in motion an exoskeleton (1).
